# EUROPÄISCHE PATENTANMELDUNG

(11) **EP 4 383 953 A1**
(43) Veröffentlichungstag der Anmeldung: **12.06.2024**
(21) Anmeldenummer: 23169414.2
(22) Anmeldetag: 24.04.2023
(51) Int. Cl.: H05H 1/00, H05H 1/24, A61L 9/22, F24F 8/30

(54) **IONENSENSOR FÜR DIE REGULATION DER LEISTUNG EINES PLASMAGENERATORS**

(30) Priorität: 08.12.2022 EP 22212242
(71) Anmelder: DBD Plasma GmbH, 37085 Göttingen (DE)
(72) Erfinder: Damm, Roland, 37160 Ebergötzen (DE); Gredner, Alexander, 37133 Friedland (DE); Haese, Christian, 37130 Gleichen (DE); Haese, Jürgen, 37130 Gleichen (DE)
(74) Vertreter: Hertin und Partner Rechts- und Patentanwälte PartG mbB

(57) **Zusammenfassung**

Die Erfindung betrifft bevorzugt eine Vorrichtung zur Erzeugung von Ionen in einem Luftstrom umfassend eine Spannungsquelle und einen Plasmagenerator, wobei sich der Plasmagenerator in dem Luftstrom befindet. Die Vorrichtung weist einen lonensensor und eine Auswerteeinheit auf, wobei die Auswerteeinheit dazu konfiguriert ist in Abhängigkeit eines Messsignals des lonensensors eine Leistung der Spannungsquelle zu regulieren.

## Beschreibung

Die Erfindung betrifft bevorzugt eine Vorrichtung zur Erzeugung von Ionen in einem Luftstrom umfassend eine Spannungsquelle und einen Plasmagenerator, wobei sich der Plasmagenerator in dem Luftstrom befindet. Die Vorrichtung weist einen lonensensor und eine Auswerteeinheit auf, wobei die Auswerteeinheit dazu konfiguriert ist in Abhängigkeit eines Messsignals des lonensensors eine Leistung der Spannungsquelle zu regulieren.

### Hintergrund und Stand der Technik

Die Erfindung betrifft das Gebiet der Erzeugung von Plasma, vorzugsweise von kaltem Plasma. Hierbei bezeichnet kaltes Plasma vorzugsweise ein teilionisiertes Gas, welches bei Zimmertemperatur und Atmosphärendruck (oder Niederdruck) bereitgestellt wird.

Durch eine Kontrolle der Konzentration und Zusammensetzung der Plasmakomponenten bzw. Ionen lassen sich die reaktiven Eigenschaften des Plasmas für verschiedenste Anwendungen anpassen.

Beispielsweise ist es bekannt kaltes Atmosphärendruckplasma zur Oberflächenbehandlung und Oberflächenmodifikation von Werkstoffen einzusetzen. So könnten vorteilhaft z.B. Hafteigenschaften in der Aufbau- und Verbindungstechnik optimiert werden oder die Notwendigkeit einer chemische Vorbehandlung von Werkstoffen reduziert werden.

Die Erzeugung kalten Plasma kann weiterhin zur Desinfektion strömender Luft, umströmter Körperbereiche oder ganzer Raumbereiche genutzt. Hierbei werden Plasmageneratoren genutzt, um einen Strom von Ionen zu erzeugen, welche als reaktive Spezies den Zellen von potenziellen Krankheitserregern auf der Haut reagieren, sodass diese durch Membran- und/oder DNA-Schäden, abgetötet werden.

Ein Händetrockner, welcher das Prinzip der Erzeugung eines reaktiven lonenstroms durch einen Plasmagenerator zur Desinfektion von Körperpartien nutzt, ist beispielsweise aus der WO 2022/063446 A1 und WO 2022/248097 A1 bekannt.

Um für die verschiedenen Anwendungsgebiete die gewünschte Wirkung zuverlässig zu erzielen, ist es notwendig die reaktiven Eigenschaften des Plasmas präzise zu kontrollieren und vorzugsweise einen konstanten lonenstrom mit den gewünschten Eigenschaften zu gewährleisten.

Diesbezüglich besteht bei bekannten Vorrichtung zur Erzeugung eines lonenstroms durch eine Plasmaquelle bedarf an Verbesserung.

### Aufgabe der Erfindung

Aufgabe der vorliegenden Erfindung war es eine Vorrichtung zur Erzeugung eines lonenstroms durch einen Plasmagenerator bereitzustellen, welche sich durch eine präzise Kontrolle der reaktiven Eigenschaften des Plasmas auszeichnet und vorzugsweise eine konstante Zusammensetzung und/oder Konzentration von Ionen in einem Luftstrom sicherstellt.

### Zusammenfassung der Erfindung

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

In einer bevorzugten Ausführungsform betrifft die Erfindung eine Vorrichtung zur Erzeugung von Ionen in einem Luftstrom umfassend eine Spannungsquelle und einen Plasmagenerator, wobei sich der Plasmagenerator in dem Luftstrom befindet, dadurch gekennzeichnet, dass die Vorrichtung einen lonensensor und eine Auswerteeinheit aufweist, wobei die Auswerteeinheit dazu konfiguriert ist in Abhängigkeit eines Messsignals des lonensensors eine Leistung der Spannungsquelle zu regulieren.

Durch die Bereitstellung eines lonensensor lassen sich vorteilhaft auf besonders einfache und präzise Weise die reaktiven Eigenschaften des Plasmas regulieren. Hierbei ist es besonders bevorzugt, dass der lonensensor in Strömungsrichtung des Luftstroms hinter dem Plasmagenerator angeordnet vorliegt.

Bei bekannten Vorrichtungen des Standes der Technik kann es aus unterschiedlichsten Gründen zu Schwankungen in Bezug auf die Konzentration und/oder Zusammensetzung der durch den Plasmageneratoren erzeugten Ionen kommen. Beispielsweise kann es im Bereich des Plasmagenerators aufgrund der Änderungen von Umgebungsparameter (Luftzusammensetzung, Druck- oder Temperaturschwankungen) zu Fluktuation in der Erzeugung des Plasma kommen. Auch Spannungsdurchschläge können sich nachteilig auf die Bereitstellung eines Plasmas mit gewünschten Eigenschaften auswirken.

Ebenso ist es bekannt, dass in Abhängigkeit der Leistung mit der ein Plasmagenerator betrieben wird sich die Zusammensetzung des Plasmas ändern kann. Beispielsweise kann es passieren, dass im Falle der Erzeugung eines kalten atmosphärischen Plasmas bei einer dielektrischen Barriereentladung durch Erhöhung der Leistung einer Spannungsquelle mehr Ozon produziert wird. Zwar weist, wie im Folgenden noch näher erläutert, Ozon vorteilhafte Eigenschaften zur Keimtötung auf, welche zur Desinfizierung genutzt werden können. Aufgrund der starken oxidierenden Wirkung und möglichen Gesundheitsrisiken bei hohen Konzentrationen, ist die zulässige Ozonmenge jedoch reguliert, sodass eine Überschreitung definierter Grenzwerte zu vermeiden ist.

Durch die Implementierung eines lonensensor kann vorteilhaft auf zuverlässige Weise unerwünschten Fluktuation in der Plasmakonzentration und/oder -zusammensetzung entgegengewirkt werden, in dem eine erfindungsgemäß vorgesehene Auswerteeinheit in Abhängigkeit eines Messsignals die Leistungszufuhr zur Spannungsquelle und mithin die Leistungszufuhr zum Plasmagenerator regelt.

Der lonensensor kann bevorzugt im Wesentlichen aus einem Messfühler aus einem leitfähigen Material bestehen, wobei in Abhängigkeit, der auf den Messfühler auftretenden Ionen, eine Messstrom als Messsignal erzeugt wird.

Das Messprinzip kann hierbei vorzugsweise ausnutzen, dass die vom Plasmagenerator erzeugen Ionen durch die Luftströmung zu dem Messfühler transportiert werden. Insbesondere bei einer gepulsten Ansteuerung des Plasmagenerators führt dies in Abhängigkeit des Luftstroms vorzugsweise zu einem Auftreffen von Ionen auf dem Messfühler, wobei vorzugweise Ionen eines Vorzeichens bevorzugt auftreffen. Hierdurch wird vorzugsweise entweder ein positiver Strom (beim vermehrten Auftreffen positiver Ionen) oder ein negativer Strom (bei einem vermehrten Auftreffen negativer Ionen) erzeugt. Die Stromstärke des erzeugten Stroms dient als Messsignal auf dessen Basis unter Zwischenschaltung einer Auswerteeinheit eine Regulation der Leistung der Spannungsquelle und mithin des Plasmagenerators vorgenommen wird.

So können beispielsweise für das Messsignal in Form eines Stroms ein Normalbereich in Form zweier Schwellwerte festgelegt. Bei Überschreiten eines ersten Schwellwertes kann die Auswerteeinheit in Abhängigkeit der Überschreitung des Messsignals beispielsweise die Leistung der Spannungsquelle (z.B. in Form eines Hochstromgenerators) durch Reduktion der Frequenz erzeugter Hochspannungspulse reduzieren. Bei Überschreiten eines zweiten Schwellwertes kann bevorzugt sein, die Leistung der Spannungsquelle und mithin des Plasmagenerators nahezu auf Null zu führen. Hierdurch kann auf einfache und zuverlässige Weise ein Gleichgewichtszustand erreicht werden, welcher den Betrieb des Plasmagenerators in einem definierten Arbeitsbereich gewährleistet.

Durch die beschriebene Regelung der Spannungsquelle bzw. eines damit betriebenen Plasmagenerators können ausgezeichnete Ergebnisse in Bezug auf die Stabilität eines lonenstroms erzielt werden, wobei insbesondere auch eine Konzentration und/oder Zusammensetzung der Ionen hochpräzise gesteuert werden kann.

Die erfindungsgemäße Regelung eines lonenstroms eines Plasmagenerators mittels des lonensensor ist vorteilhaft auf eine Vielzahl von Plasmaarten und Plasmageneratoren anwendbar.

Bevorzugt kann die Vorrichtung insbesondere zur präziseren Regulation eines sogenannten kalten Plasmas zum Einsatz kommen, wobei bevorzugt das physikalische Prinzip der dielektrischen Barriereentladung zum Tragen kommt.

Bei einer dielektrische Barriereentladung wird ein Plasma bevorzugt durch das Anlegen einer hohen Spannung zwischen zwei Elektroden erzeugt, wobei mindestens eine der Elektroden durch ein Dielektrikum isoliert ist.

Durch die Verwendung der Isolierung wird vorzugsweise das Entstehen einer Bogenentladung verhindert. Stattdessen bilden sich bevorzugt viele feine Plasmafilamente zwischen den Elektroden aus, die jedoch bevorzugt nur eine geringe Lebensdauer im Bereich von einigen Nanosekunden haben. Grund dafür ist die Ansammlung der Ladungsträger auf der Oberfläche des Dielektrikums, die ein Gegenfeld zu der von außen angelegten Spannung erzeugen, sodass die Entladung wieder erlischt. Daher wird typischerweise eine Spannungsquelle in einem Pulsbetrieb verwandt bei dem beispielsweise Hochspannungspulse mit einer Kilohertz Frequenz (1 kHz bis 1000 kHz) betrieben werden.

Die dielektrische Barriereentladung kann sowohl im Niederdruck als auch bei Atmosphärendruck erzeugt werden, wobei sich deren besonderen Eigenschaften vor allem bei Atmosphärendruck entfalten. Aufgrund der großen Masse der Ionen im Vergleich zu den Elektroden, können die schweren Ionen weit weniger Energie aus dem Wechselfeld aufnehmen als die leichteren und schnelleren Elektronen. Es handelt sich daher bevorzugt um ein nichtthermisches "kaltes" Plasma, bei dem die Elektronen eine hohe Temperatur besitzen, das Neutralgas und die Ionen jedoch bei etwa Raumtemperatur vorliegen. Dadurch lassen sich auch temperaturempfindliche Werkstoffe, Materialien oder auch biologische Systeme, wie beispielsweise die menschliche Haut mit diesem Entladungstyp behandeln.

Eine dielektrische Barriereentladung zur Erzeugung eines kalten Plasmas kann für vielfältigste Anwendungen eingesetzt werden, wozu auch eine Luft- und Abwasserbehandlung, eine Entkeimung als auch eine Aktivierung, Reinigung, Ätzung und Beschichtung von Oberflächen verschiedenster Werkstoffe zählen. Ein großer Vorteil der dielektrischen Barriereentladung ist es, dass sie durch flexible Anordnung der Elektroden- oder Dielektrikums in verschiedene Formen und Größen hergestellt und somit gut an eine bestimmte Anwendung angepasst werden kann.

Die beschriebene Regelung der Leistung einer Spannungsquelle bzw. des Plasmagenerators mittels eines lonensensor führt vorteilhaft zu einer besonders stabilen Erzeugung eines kalten Plasmas bei atmosphärischen Druck.

Die Regelung lässt sich vorteilhaft auf verschiedenste Plasma-Jet bzw. -Remote-Systeme anwenden, welche die Entladung in einer externen Kavität erzeugen. Ebenso können Systeme die eine Entladung (Plasma) ausblasen oder auch Systeme mit direkt abgeführten Abgasströmen eines Plasmas von der beschriebenen Regelung profitieren.

In einer besonders bevorzugten Ausführungsform ist die Vorrichtung zum Desinfizieren von Körperbereichen eingerichtet, beispielweise zum Desinfizieren einer menschlichen Haut. Beispielsweise kann die Vorrichtung als eine Handtrockner ausgeführt sein mit einem Lüfter zur Erzeugung des Luftstroms, optional mit einem oder mehreren Heizelemente.

Ein Luftstrom bezeichnet im Sinne der Erfindung bevorzugt die Bewegung von Luft. Luft verhält sich fluidisch, d. h. Partikel fließen von Natur aus von Bereichen mit höherem Druck zu solchen, in denen der Druck niedriger ist. Vorzugsweise wird der Luftstrom von einem Lüfter erzeugt, welcher Bestandteil der Vorrichtung sein kann.

Ein Lüfter kann im Sinne der Erfindung der Erfindung auch als Gebläse bezeichnet werden und meint bevorzugt eine angetriebene Strömungsmaschine, die ein gasförmiges Medium, bevorzugt Luft, fördert. Dazu kann der Lüfter ein axial oder radial durchströmtes Laufrad aufweisen, das bevorzugt in einem Gehäuse der erfindungsgemäßen Vorrichtung rotiert. Im Stand der Technik sind verschiedene Bauformen von Lüftern bekannt, die in der erfindungsgemäßen Vorrichtung eingesetzt werden können. Hierzu gehören ohne Beschränkung Axiallüfter, Radiallüfter, Diagonallüfter oder auch Tangentiallüfter.

Bei der Verwendung der Vorrichtung zum Desinfizieren von Körperbereichen wird bevorzugt ausgenutzt, dass im Plasma reaktive Spezies enthalten sind, bevorzugt reaktive Sauerstoff- und Stickstoffspezies. Reaktive Spezies zeichnen sich dadurch aus, dass sie hochreaktiv sind und eine desinfizierende Wirkung entfalten können. Vorzugsweise sind die reaktiven Sauerstoff- und Stickstoffspezies derart langlebig sind, dass sie auch außerhalb des Entladungsbereichs wirken können. Insbesondere sind sie über einen Zeitraum stabil, dass sie von der umströmten Luft zu desinfizierende Körperbereiche, insbesondere den Händen, und/oder der Umgebungsluft befördert werden können.

Reaktive Sauerstoffspezies (*englisch reactive oxygen species,* ROS) werden auch vereinfachend als "Sauerstoffradikale" bezeichnet, während reaktive Stickstoffspezies (*englisch reactive nitrogen species,* RNS) reaktive Stickstoffverbindungen bezeichnen. Bei den reaktiven Sauerstoffspezies kann es sich um Singulett-Sauerstoff ¹O₂ und Hyperoxid-Anionen handeln, wobei die reaktiven Stickstoffspezies Stickstoffmonoxid umfassen können, wobei das Stickstoffmonoxid mit den Hyperoxid-Anionen zu Peroxinitriten ONOO⁻ und ONOOH reagiert.

Weiterhin kann im Plasma auch Ozon O₃ entstehen, welches eine starke Oxidationswirkung aufweist und auch bei Raumtemperatur instabil ist. Bei Zersetzung des Moleküls entsteht atomaren Sauerstoff, der selbst hoch reaktiv ist und oxidativ wirkt. Ozon ist damit als sogenannter "aktiver Sauerstoff" quasi ein Träger dieses reaktiven atomaren Sauerstoffs. Durch die hohe Reaktivität entfaltet Ozon eine desinfizierende Wirkung auf organische Verbindungen, zum Beispiel Bakterien oder Viren. Vorteilhaft sind die durch das Ozon zersetzten Verbindungen biologisch abbaubar und auch das nach der Zersetzungsreaktion unverbrauchte Ozon zerfällt selbstständig, wobei lediglich Sauerstoff als Zersetzungsprodukt verbleibt.

In einer bevorzugten Ausführungsform umfasst der Plasmagenerator eine Elektrodenpaar, wobei mindestens eine der Elektroden durch ein Dielektrikum isoliert ist und wobei durch Anlegen einer Spannung an das Elektrodenpaar durch die Spannungsquelle ein Plasma erzeugt wird.

Die Anordnung eignet sich insbesondere zur Erzeugung eines kalten Plasmas durch eine dielektrischen Barriereentladung.

Ein Plasma bezeichnet im Sinne der Erfindung bevorzugt ein Teilchengemisch aus Ionen, freien Elektronen und meist auch Atomen und/oder Molekülen. Ein Plasma enthält also freie Ladungsträger (Ionen). Der Ionisationsgrad eines Plasmas kann weniger als 1 % betragen, aber auch 100 % (vollständige lonisation).

Im Falle der dielektrischen Barriereentladung ist das Plasma, bevorzugt das schon erwähnte kalte Plasma, nichtthermisch, d. h. das Plasma befindet sich nicht im thermischen Gleichgewicht. Konkret bedeutet dies, dass sich die Temperaturen der enthaltenen Teilchensorten signifikant unterscheiden.

In der dielektrischen Barriereentladung wird trotz eines normalen Luftdrucks ein Plasma weit ab vom thermischen Gleichgewicht erzeugt. Durch hochfrequente Spannungspulse bilden sich bevorzugt mit jedem Spannungspuls winzige Entladungskanäle. Diese Entladungskanäle werden im Stand der Technik auch als "Streamer" bezeichnet. Da die Entladungskanäle nur einen Bruchteil des gesamten Entladungsvolumens ausmachen und die Zeitdauer der Entladung durch die kapazitive Kopplung stark begrenzt ist, bleibt die mittlere Gastemperatur in der Entladung niedrig und damit in der Nähe der Raumtemperatur.

In einer bevorzugten Ausführungsform umfasst der Plasmagenerator mindestens ein Plasmastab, welcher ein dielektrisches Rohr mit einem elektrisch leitenden Kern innerhalb des dielektrischen Rohres umfasst, wobei das dielektrische Rohr auf einer Außenseite einen über zu Windungen gewickelten Draht aufweist und wobei der elektrisch leitende Kern mit dem auf der Außenseite gewickelten Draht ein Elektrodenpaar bildet, welches bei Anlegen einer Spannung ein Plasma erzeugt. Das dielektrische Rohr mit dem Elektrodenpaar wird im Sinne der Erfindung auch als Plasmastab bezeichnet.

Die Ausführungsform hat sich als eine besonders effektive Anordnung zur Erzeugung eines kalten Plasma, über eine große Wirkfläche erwiesen. Besonders bevorzugt findet ein solcher Plasmagenerator einen Einsatz für die desinfizierende Wirkung auf Körperbereiche.

Das dielektrische Rohr umfasst bevorzugt zu diesem Zweck ein dielektrisches Material, welches eine elektrischen Strom nicht oder nur sehr schwach leitet. Beispielhafte Materialien für das dielektrische Rohr umfassen Polyethylen, Polytetrafluorethylen (PTFE), Keramik, beispielsweise Steatit und/oder Aluminiumoxid, Glimmer und/oder Glas, u.a. Borsilikatglas.

In Bezug auf eine Dimensionierung kann das dielektrische Rohr vorzugsweise eine Länge von einem 4-fachen bis zu einem 30-fachen des Außendurchmessers aufweisen.

Entlang einer Außenseite des dielektrische Rohr wird der Draht zu Windungen gewickelt. Der elektrisch leitende Kern innerhalb und der zu Windungen gewickelte Draht außerhalb des dielektrischen Rohres bilden das Elektrodenpaar.

Der elektrisch leitfähige Kern kann vorzugweise ein Volldraht oder ein Rohr sein, der vorzugsweise von seinen Ausmaßen so ausgebildet ist, dass er in das dielektrische Rohr hineinpasst. Beispielsweise kann der elektrisch leitende Kern in Form eines Rohres ausgebildet werden, welches aus einem Drahtgeflecht besteht und von den Ausmaßen derart ausgebildet ist, dass der elektrisch leitenden Kern in das dielektrische Rohr hineinpasst.

Vorzugsweise ist der Draht auf der Außenseite des dielektrischen Rohres doppelt und gegenläufig gewickelt. Damit wird die Induktivität, die diese Wicklung haben könnte, kompensiert. Durch die doppelte Wicklung wird ein Aufbau ähnlich wie bei einer Koaxialleitung erreicht, die keine elektromagnetischen Strahlen aussendet. Stattdessen können die stromdurchflossenen Wicklungen effektiv zur Erzeugung des Plasmas dienen. Um das dielektrische Rohr gelangt der Luftstrom durch den Lüfter bedingt vorzugsweise in Querrichtung.

Durch das Anlegen einer Spannung, bevorzugt eine Wechselspannung bzw. Hochspannungspulse, entsteht vorzugsweise die dielektrische Barriereentladung und somit das Plasma entlang der Oberfläche des dielektrischen Rohres.

Der Draht kann bevorzugt einen Durchmesser zwischen 0,1 mm und 1 mm, bevorzugt zwischen 0,2 mm und 0,4 mm, aufweisen. Vorzugsweise weist das dielektrische Rohr eine glatte Oberfläche auf. Eine glatte Oberfläche meint insbesondere ein Mittenrauwert (arithmetisches Mittel der betragsmäßigen Abweichung von einer Mittellinie der Oberfläche) zwischen 0 µm und 500 µm, bevorzugt 0 und 50 µm oder besonders bevorzugt bis zu 5 µm, bis zu 1 µm aufweist.

Die Verwendung ein dünnen Drahtes auf einer dielektrischen Rohres ist vorteilhaft für eine glatten Umströmung der Luft, wobei eine Grenzschicht entlang des dielektrischen Rohres relativ dünn ist und selbst kaum strömt. Die genannten Ausmaße wirken sich insbesondere für die Ausbildung einer dünnen Grenzschicht vorteilhaft aus. Dieses ist insofern vorteilhaft, als dass die Luft in dieser Grenzschicht kaum strömt. Daher müssen Plasmakomponenten, beispielsweise reaktiven Spezies, durch diese Schicht hindurch diffundieren. Des Weiteren wird diese Grenzschicht an wenigen Stellen durch den gewickelten Draht gestört. Dies bringt Turbulenzen mit sich, was von Vorteil ist, da die Durchmischung der Plasmakomponenten mit der umströmten Luft erheblich verbessert wird und so diese als Ionen im Luftstrom für die weitere Anwendung (beispielsweise zur Desinfektion) zum Einsatz kommen können.

Die Umwicklung des dielektrischen Rohres durch einen dünnen Draht erlaubt zudem eine besonders zuverlässige Ausbildung eines kalten Plasmas auch bei Raumtemperatur über eine große Wirkfläche zu verteilen. Ein Grund dafür liegt darin, dass das Plasma in der Regel dort leichter zündet, wo die Elektrode oder das Dielektrikum wärmer ist. Wenn die angelegte Spannung nur geringfügig über der zum Zünden notwendigen Spannung liegt, führt das dazu, dass das Plasma zumeist nur an den Stellen zündet, an denen die Temperatur erhöht ist. An anderen Stellen zündet es eventuell gar nicht mehr. Effektive Abhilfe kann geschaffen werden, indem die Spannung erhöht wird, was allerdings die Leistung steigert, oder die Frequenz herabgesetzt wird, was wiederrum eine Vergrößerung der evtl. vorhandenen Transformatoren erfordert.

Durch die Entzündung des Plasmas entlang einer dünnen Linie der Drahtwicklung lässt sich die Leistung trotz großer Frequenz und großer Spannung auf ein gewünschtes Maß reduzieren, ohne die Länge des Rohres zu verringern. Somit kann vorteilhafterweise die Erzeugung von Plasmakomponenten auf eine größere Strecke bzw. auf eine größere Fläche verteilt werden und ein größeres Strömungsprofil gleichmäßig mit Ionen versorgt werden.

Durch das Feedback des lonensensors, welche mittels einer Auswerteeinheit die Leistung der Spannungsquelle reguliert, werden vorteilhaft auch über eine große Wirkfläche äußerst stabile Bedingungen für die Erzeugung des Plasmas gewährleistet.

Der Draht kann vorteilhafterweise mit nahezu beliebigen Windungsabstand gewickelt werden. Bevorzugt können die Windungen des Drahtes einen Abstand zueinander bis zu einem 10-fachen des Außendurchmessers des dielektrischen Rohres, bevorzugt zwischen dem 1-fachen Außendurchmesser und dem 10-fachen Außendurchmesser, mehr bevorzugt zwischen dem 1-fachen Außendurchmesser und dem 5-fachen Außendurchmesser, besonders bevorzugt zwischen dem 2-fachen und dem 4-fachen Außendurchmesser, aufweisen. Der Abstand der Windungen meint bevorzugt die mittlere Distanz zwischen Windungen des Drahtes in einer Wicklungsrichtung. Bevorzugt kann der Abstand zwischen Windungen des Drahtes entlang der Längsachse auf einer Außenkontur des Drahtes gemessen werden. Im Falle einer Wicklung des Drahtes mit vier Windungen in einer Wicklungsrichtung über eine Länge von 80 mm beträgt der Abstand beispielsweise ca. 20 mm.

In einer Ausführungsform ist die Vorrichtung dadurch gekennzeichnet, dass der Plasmagenerator ein Array von mehreren Plasmastäben umfasst. Im Sinne der Erfindung bezeichnet ein Array eine Anordnungsstruktur des Plasmagenerators bzw. der Plasmastäbe. Bevorzugt können mehrere Plasmastäbe, welche ein Array bilden, den Plasmagenerator darstellen.

Vorzugsweise ist das Array ein-, zwei-, und/oder dreidimensional ausgestaltet. Dabei sind die Plasmastäbe ein-, zwei und/oder dreidimensional bevorzugt durch Parallelschaltungen und/oder Serienschaltung miteinander verschaltet.

Im Falle der Ausbildung eines Plasmagenerators als Array umfassend eine Mehrzahl von Plasmastäben kann es bevorzugt sein, jedem Plasmastab ein lonensensor zur Messung der erzeugten Ionen zuzuordnen. Ebenso kann es bevorzugt sein, lediglich einen lonensensor für den gesamten Array bereitzustellen.

Vorzugsweise erfolgt ein Betrieb der Mehrzahl der Plasmastäbe mittels einer Spannungsquelle, vorzugsweise unter Zuhilfenahme einer Steuereinheit wie hierin beschrieben. Weiterhin ist es auch für die Ausführungsform eines Arrays bevorzugt, dass eine Auswerteeinheit die Messsignale der einen oder mehreren lonensensoren aufnimmt, beispielsweise in Form eines Summensignals, und auf deren Basis eine Reduktion der Leistung der Spannungsquelle und mithin des dadurch angetriebenen Plasmagenerators umfassend eine Mehrzahl von Plasmastäben reduziert.

In einer bevorzugten Ausführungsform ist die Spannungsquelle einen Transformator, vorzugsweise einen Hochspannungstransformator, umfasst, wobei die Spannungsquelle besonders bevorzugt zur Erzeugung asymmetrischer Hochspannungspulse konfiguriert ist.

Zur Ansteuerung der Spannungsquelle weist die Vorrichtung vorzugsweise eine Steuereinheit auf. Eine Steuereinheit kann insbesondere eine elektronische Schaltungen umfassen, wozu ohne Beschränkung eine integrierte Schaltung (IC), ein Anwendungsspezifische integrierte Schaltungen (ASIC), eine programmierbare logische Schaltung (PLD), ein Field Programmable Gate Array (FPGA), einen Mikroprozessor, einen Mikrocomputer, eine speicherprogrammierbare Steuerung und/oder eine sonstige elektronische, bevorzugt programmierbare, Schaltung gehören.

Ein Transformator umfasst bevorzugt zwei oder mehr Spulen, die sich meist aus isoliertem Kupferdraht gewickelt sind und sich an einem gemeinsamen Magnetkern befinden. Der Transformator wandelt vorzugsweise eine Eingangswechselspannung, die an einer der Spulen angelegt ist, in eine Ausgangswechselspannung um, die an der anderen Spule abgegriffen werden kann.

Besonders bevorzugt umfasst die Spannungsquelle einen Hochspannungstransformator, welcher für eine Erzeugung einer asymmetrischer Hochspannungspulse ausgelegt ist. Zu diesem Zweck ist es bevorzugt, dass die Steuereinheit ein entsprechendes Signal zur Ansteuerung des Hochspannungstransformator bereitstellt.

Bevorzugt kann die Steuereinheit dafür konfiguriert sein, Hochspannungspulse mit verschiedener Amplitude oder Wiederholrate zu generieren, um durch den Hochspannungstransformator eine abklingenden Wechselspannung bereitzustellen. Bevorzugte Spannungspulse können beispielsweise die Form eines abklingenden Sinus aufweisen. Hierbei kann die Spannung bevorzugt im Wesentlichen aus einer positiven und einer ähnlich großen negativen Halbwelle zusammengesetzt sein, gefolgt von mehreren Nachschwingern. Für die Frequenz einer abklingenden Wechselspannung können beispielsweise zwischen 100 kHz (Kilohertz) und 1000 kHz, vorzugsweise 500 kHz bis 1000 kHz eingesetzt werden, wobei eine Wiederholrate der Spannungspulse beispielsweise aus einem Bereich von 100 Hz bis 10 kHz ausgewählt sein kann. Die Spannungspulse können Spitze-Spitze von mindestens 1 kV, mindestens 5 kV, 10kV oder auch mehr aufweisen.

Besonders bevorzugt ist die Dauer der Pulse relativ kurz, beispielsweise weisen können die Pulse vorzugsweise eine Dauer (FWHM *full width at half maximum*) im Mikrosekundenbereich oder auch weniger aufweisen.

Besonders bevorzugt erfolgt eine Erzeugung einer asymmetrischen Hochspannung: Hierbei folgt auf eine kurze große positive (oder negative) Spannungsspitze eine relativ langen Zeit geringer negativer (oder positiver) Spannung.

Ein derartiges asymmetrisches Wechselfeld erlaubt eine besonders gute Trennung positiver und negativer Ionen im Luftstrom zur Erzeugung eines Stromsignals bei Ausführungsform des lonensensors als ein Messfühler.

In einer bevorzugten Ausführungsform umfasst der lonensensor einen Messfühler aus einem leitfähigen Material, wobei der Messfühler vorzugsweise in Form eines Sensorstabes vorliegt und das leitfähige Material vorzugsweise ein Metall ist.

Das Messprinzips des bevorzugten lonensensor basiert vorzugsweise auf einem Einfangen der Ionen. Hierbei umfasst der lonensensor vorzugweise einen Messfühler aus einem leitfähigen Material, beispielsweise Metall. Der Messfühler kann unterschiedlichste Form aufweisen und beispielsweise als Messplättchen oder Messstab ausgebildet sein. Besonders bevorzugt liegt der lonensensor in Form eines Mess- bzw. Sensorstabes aus Metall vor.

Der lonensensor ist vorzugweise in einer geringen Entfernung zum Plasmagenerator in Strömungsrichtung hinter diesem angebracht. Hierbei meint eine geringe Entfernung vorzugsweise eine Entfernung von weniger als 50 mm, weniger als 20 mm, weniger als 10 mm oder weniger. Bevorzugt ist der Abstand des lonensensors von der Plasmaquelle jedoch mindestens 1 mm, bevorzugt mindestens 5 mm.

Durch den Abstand zwischen Plasmagenerator und lonensensor kann vorzugsweise die Feldstärke eines elektrischen Feldes beeinflusst werden, welche eine Driftbewegung der Ionen bewirkt. In der Regel werden die Ionen sich schneller zum lonensensor bewegen, je größer die Feldstärke. Die Feldstärke sollte jedoch vorzugsweise auch nicht derart groß sein, dass sich ein Plasma zwischen einem Metallstab als lonensensor und dem Plasmagenerator ausbilden kann. Die bevorzugt genannten Parametergrenzen führen vorteilhaft zu einer besonders hohen Sicherheit vor etwaigen elektrischen Überschlägen von der Plasmaquelle zum lonensensor und erlauben gleichzeitig eine zuverlässige Messung des lonenstroms.

Vorzugweise wird durch die Spannungsquelle ein asymmetrisches Wechselfeld erzeugt, welches eine Trennung von positiven und negativen Ionen zur Detektion durch den lonensensor begünstigt. Hierbei kann es bevorzugt sein, dass durch die asymmetrischen Hochspannungspulse ein elektrisches Feld erzeugt wird, welches in einer Richtung stärker ist, als in einer anderen Richtung. So kann es beispielsweise bevorzugt sein, dass kurze große positive (oder negative) Spannungsspitzen erzeugt werden, mit der sich die Ionen mit einem bevorzugten Vorzeichen (positiv oder negativ) schneller als die Luftströmung in Richtung des lonensensors bewegen. Die Spannungsspitzen sind gefolgt von einer im Vergleich langen Zeit geringer negativer (oder geringer positiver) Spannung, bei der die Bewegung der Ionen durch die Luftströmung dominiert wird und die Ionen (mit einem umgekehrten Vorzeichen) deutlich seltener auf den lonensensor treffen.

Hierdurch kann in Abhängigkeit der Vorzugsladung der Ionen ein positiver oder negativer Strom (sogenannter lonenstrom) erzeugt werden.

In einer bevorzugten Ausführungsform ist das Messsignal des lonensensors mithin ein Strom (lonenstrom).

Vorteilhaft ist für einen derartigen lonensensor kein zusätzliches Anlegen einer Spannung am lonensensor selbst notwendig. Eine gängige und bekannte Methode der lonenmessung in Luft basiert auf dem Prinzip der Gerdien-Röhre. Hierbei handelt es sich um eine Messröhre mit einem Rohr und einem inneren koaxialen Metallstab oder Draht koaxial. Zwischen dem Draht bzw. Metallstab und dem Rohr wird eine Gleichspannung angelegt, während durch das Rohr Luft strömt. Anhand des gemessenen Stroms kann auf die lonendichte in der strömenden Luft geschlossen werden.

Ein derartiges Anlegen einer Gleichspannung ist bei einer Ausführungsform des lonensensor als Messfühler aus einem leitfähigen Material vorteilhaft nicht notwendig. Stattdessen kann ausgenutzt werden, dass in der Nähe des Plasmagenerators eine lonendichte haben, welche ein Vielfaches von dem beträgt, was sonst in üblichen Wohnumgebungen.

Unter Ausnutzung der pulsierende Hochspannung mit asymmetrischen Hochspannungspulsen kann hierbei auch ohne Anlegen einer externen Spannung am Messfühler ein lonenstrom als Messsignal für die Qualität des Plasmas abgegriffen werden. In Vorzugsformen der asymmetrischen Hochspannungspulsen ist die mittlere Spannung gleich Null, allerdings sind die Beträge von negativen und positiven Spannungsspitzen stark unterschiedlich. Das führt im Zusammenhang mit einer Luftströmung wie erläutert dazu, dass Ionen eines Vorzeichens (positive oder negative Ladung) besonders gut den lonensensor bzw. Messfühler erreichen, während die Ionen des anderen Vorzeichens infolge eines schwachen Feldes vorzugsweise von der Luftströmung weggerissen und den lonensensor nicht oder in vermindertem Maße erreichen.

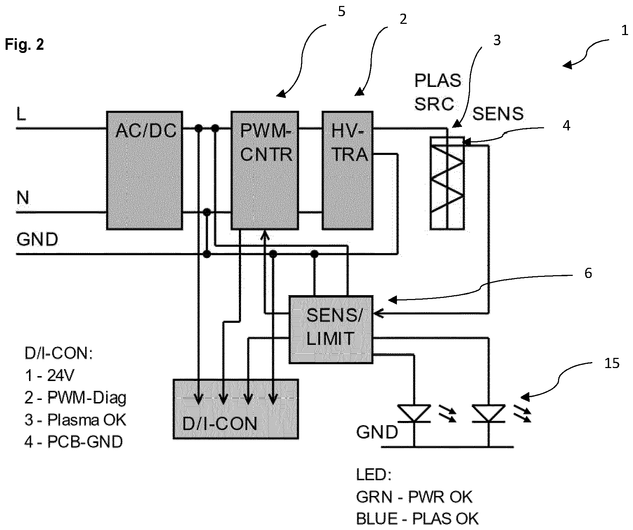

In bevorzugten Ausführungsformen kann auf die Bereitstellung eines zum Plasmagenerators separaten Messfühlers bzw. Metallstabs verzichtet werden. Stattdessen kann vorzugsweise eine Gegenelektroden des Plasmagenerators unmittelbar als Messfühler bzw. lonensensor verwendet werden. Zudem diesem Zweck kann es bevorzugt sein die Gegenelektrode an ein Massepotential, vorzugsweise über einen Kondensator zu koppeln (vgl. Fig. 2). Dadurch schwingt die Spannung nur noch wenig, während der lonenstrom als Gleichstrom gemessen werden kann. Besonders bevorzugt kann zwischen dem lonensensor und der Messelektronik bzw. Auswerteeinheit einen Tiefpassfilter installiert werden, sodass die Spannungsschwankungen durch die Hochspannung nicht auf die Elektronik der Auswerteeinheit durchschlagen.

Die Auswerteeinheit ist vorzugsweise dafür konfiguriert in Abhängigkeit eines Messsignals des lonensensors eine Leistung der Spannungsquelle zu regulieren. Vorzugsweise kann eine Regulation einer Leistung der Spannungsquelle meinen, dass bei Überschreiten eines ersten Schwellwertes in Abhängigkeit der Überschreitung die Auswerteeinheit eine Leistung der Spannungsquelle und mithin des Plasmagenerators reduziert. Die Reduktion wird vorzugsweise derart in Abhängigkeit der Überschreitung verstärkt, dass bei einem Überschreiten eines zweiten Schwellwerts die Leistung der Spannungsquelle auf nahezu Null herunterreguliert würde. Nahezu Null meint bevorzugt, dass die Wiederholrate derart lang ist, dass die Erzeugung zusätzlicher Ionen durch das Plasma nahezu auf Null gefahren werden kann, ohne die Vorrichtung jedoch völlig abzuschalten, was in Bezug auf eine stabile Plasmaerzeugung nachteilig wäre.

In einer bevorzugte Ausführungsform ist die Auswerteeinheit zur Regulation der Leistung der Spannungsquelle mit einer Steuereinheit verbunden, welche eine Steuerungssignal zur Ansteuerung der Spannungsquelle bereitstellt.

Wie obig ausgeführt, kann es sich bei der Steuereinheit um eine elektronische Schaltung beispielsweise eine integrierte Schaltung (IC) oder auch einen Mikroprozessor handeln.

Bei der Auswerteeinheit kann es sich ebenfalls um eine elektronische Schaltung handeln.

In bevorzugten Ausführungsformen kann die Auswerteeinheit einen passiven Tiefpassfilter umfassen sowie optional einen analogen Spannungsverstärker. In Abhängigkeit eines Messsignals des lonensensors, beispielsweise eines lonenstroms, kann vorzugsweise bei Überschreiten eines Schwellwertes eine verstärkte Spannung erzeugt werden, welche die Leistung des Plasmagenerators über die Steuereinheit sowie Spannungsquelle herunter reguliert.

Mittels des analogen Spannungsverstärker kann die Spannung vorteilhaft derart verstärkt werden, dass eine Regelung der Steuereinheit bzw. der Spannungsquelle möglich ist. Eine Analog-Digital-Wandlung von Messwertes bzw. Messsignals des lonensensors sowohl direkt oder mit zwischengeschaltetem Verstärker ist ebenso möglich.

Die Regelung der Leistung der Spannungsquelle durch die Auswerteeinheit kann unterschiedlich konfiguriert sein.

In einer bevorzugten Ausführungsform ist die Auswerteeinheit dazu eingerichtet bei Überschreiten eines ersten Schwellwertes des Messignals des lonensensors die Leistung für die Spannungsquelle zu reduzieren, wobei es besonders bevorzugt ist, dass die Leistung für die Spannungsquelle derart in Abhängigkeit von einer Überschreitung des ersten Schwellwertes reduziert wird, dass ab einem zweiten Schwellwert des Messignals des lonensensors die Leistung der Spannungsquelle auf nahezu Null herunter reguliert wird.

Beispielsweise kann es bevorzugt sein, dass ein stabiles, kaltes Plasma mit gewünschter Konzentration oder Zusammensetzung der Ionen mit einem Messsignal des lonensensors, beispielweise einem lonenstrom, in einem bestimmten Parameter korreliert. Bei Überschreiten des Parameterbereiches, beispielsweise eines ersten Schwellwertes, kann es bevorzugt sein, dass die Auswerteeinheit dafür konfiguriert ist, die Leistung der Spannungsquelle und mithin des Plasmagenerators zu reduzieren.

Im Falle der bevorzugten Ausgestaltung einer Spannungsquelle als Hochspannungstransformator zur Erzeugung von Spannungspulsen kann es beispielsweise bevorzugt sein, dass die Reduktion der Leistung durch eine Reduktion der Frequenz der Spannungspulse vorgenommen wird. Ebenso kann es bevorzugt sein, dass die Spitze-zu-Spitze Spannungen der Pulse reduziert werden.

Durch eine derartige Reduktion der Leistung der Spannungsquelle ab einem Schwellwert kann vorteilhaft eine Übersteuerung des Plasmagenerators vermieden werden.

Vorzugweise erfolgt die Reduktion derart, dass in Abhängigkeit der Überschreitung des ersten Schwellwertes durch das Messignals des lonensensors die Reduktion verstärkt wird.

Die Reduktion wird vorzugsweise derart in Abhängigkeit der Überschreitung verstärkt, dass bei einem Überschreiten eines zweiten Schwellwerts die Leistung der Spannungsquelle auf Null herunterreguliert würde. Für ein Messsignal zwischen den beiden Schwellwerte korreliert die Reduktion der Leistung (beispielsweise durch eine Regulation der Frequenz der Spannungspulse) vorzugsweise positiv mit dem Grad der Überschreitung. Je weiter das Messsignals des lonensensors sich somit von einem festgelegten Grenzbereich entfernt desto stärker greift die Regelung der Auswerteinheit ein. Die Regelung ist vorzugsweise reversibel. D.h. sobald der lonenstrom unterhalb der definierten Schwellwerte fällt, wird die Reduktion der Leistung durch die Auswerteeinheit aufgehoben.

Vorteilhaft kann hierdurch auf einfache Weise ein Gleichgewichtszustand erreicht werden, in welchem der Plasmagenerator so eingeregelt wird, dass ein Messignal des lonensensors, beispielsweise ein lonenstrom, im durch die Schwellwerte bevorzugten Arbeitsbereich liegt.

In einer weiteren bevorzugten Ausführungsform ist die Auswerteeinheit dafür konfiguriert in Abhängigkeit eines Messignals des lonensensors eine Anzeige, vorzugweise eine optische Anzeige, an der Vorrichtung zu aktivieren. Beispielsweise kann es bevorzugt sein, dass die Vorrichtung eine oder mehrere LEDs aufweist, welche einen Betriebszustand der Vorrichtung anzeigen.

In Abhängigkeit des Messsignals des lonensensor kann die Auswerteeinheit vorzugsweise dafür konfiguriert sein, eine LEDs zum Leuchten zu bringen, beispielsweise sofern das Messsignal am lonensensor die Bereitstellung eines Plasmas im gewünschten Arbeitsbereich anzeigt.

Die Erfindung soll im Folgenden anhand von Beispielen näher erläutert und illustriert werden, ohne auf diese beschränkt zu sein.

### FIGUREN

### Kurzbeschreibung der Figuren

- **Fig. 1**: Darstellung eines Schaltbildes einer bevorzugten Ausführungsform der erfindungsgemäßen Vorrichtung
- **Fig. 2**: Darstellung eines Schaltbildes einer bevorzugten Ausführungsform der erfindungsgemäßen Vorrichtung
- **Fig. 3**: Darstellung einer bevorzugten Ausführungsform der erfindungsgemäßen Vorrichtung
- **Fig. 4**: Darstellung einer weiteren bevorzugten Ausführungsform der erfindungsgemäßen Vorrichtung

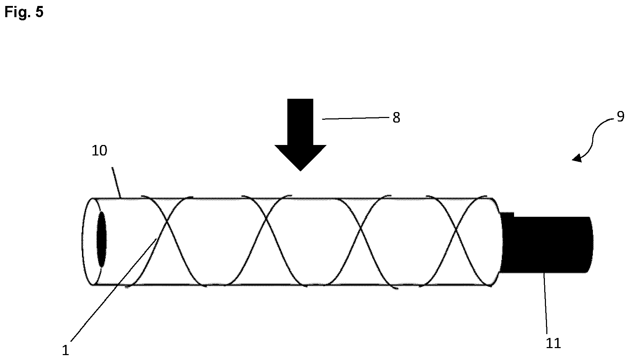

- **Fig. 5**: Darstellung einer bevorzugten Ausführungsform eines Plasmastabes eines Plasmagenerators

### Detaillierte Beschreibung der Figuren

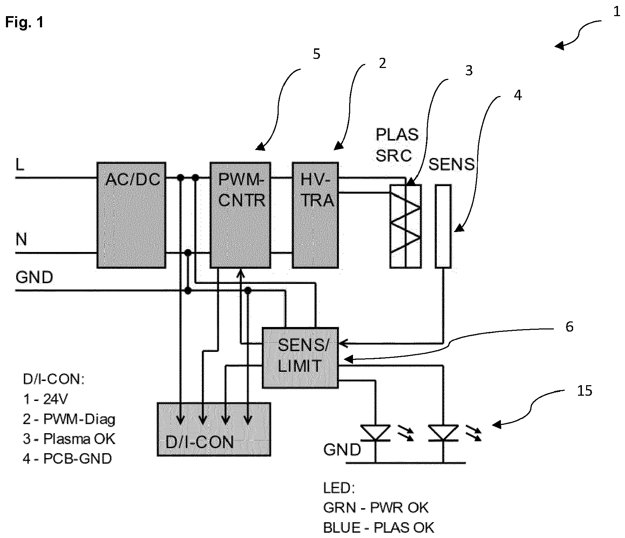

**Fig. 1** ist eine Darstellung eines Schaltbildes einer bevorzugten Ausführungsform der erfindungsgemäßen Vorrichtung **1.**

Die Vorrichtung **1** ist zur Erzeugung von Ionen in einem Luftstrom eingerichtet und umfasst zu diesem Zweck eine Spannungsquelle **3** und einen Plasmagenerator **3;** wobei sich der Plasmagenerator **3** in dem Luftstrom befindet. Die Vorrichtung **1** ist dadurch gekennzeichnet, dass diese einen lonensensor **4** und eine Auswerteeinheit **6** aufweist, wobei der lonensensor **4** in Strömungsrichtung des Luftstroms hinter dem Plasmagenerator **3** angeordnet vorliegt. Die Auswerteeinheit **6** ist dazu konfiguriert in Abhängigkeit eines Messsignals des lonensensors **4** eine Leistung der Spannungsquelle **2** zu regulieren.

In der Fig. 1 ist ein Schaltbild der bevorzugten Vorrichtung dargestellt.

Zwei Flachstecker der Platine dienen zur Stromversorgung (**L / N**). Der dritte Stecker (**GND**) dient zur Verbindung mit dem Schutzleiter und ist darüber hinaus mit der Masse der Platine verbunden. Dies soll die Sicherheit des Netzteils gegenüber einer möglichen Zerstörung durch statisch elektrische Aufladungen erhöhen.

Wenn kein Schutzleiter verfügbar ist, kann die Masse der Platine wahlweise auch mit einem Widerstand und einem Kondensator mit N verbunden werden. So wird vorzugsweise eine Ableitung von statischer Elektrizität und von hochfrequenten Störungen sichergestellt.

Die Masse der Platine ist auf dem Diagnose/Interface-Stecker (**D / I CON**) ebenfalls vorhanden. Die Komponente **AC/DC** steht für einen AC/DC Wandler, welcher aus einer Netzspannung (Wechselspannung) eine Versorgungsspannung (Gleichstrom) vorzugsweise von 24V erzeugt. In der Zeichnung nicht dargestellt, aber vorzugweise vorhanden, ist eine Versorgungsspannung für die Elektronik, welche bevorzugt 15 V beträgt.

Im Normalbetrieb übernimmt die Platine folgende Funktion: Nach einem Anlegen der Netzspannung startet der Plasmagenerator 3 sofort. Zu diesem Zweck erzeugt eine Steuereinheit **5** (**PWM-CNTR**) ein Signal zur Ansteuerung der Spannungsquelle 2 vorliegend in Form eines Hochspannungstransformators (**HV-TRA**).

Der Plasmagenerator 3 in der Figur dargestellt durch **PLAS SCR** umfasst ein Elektrodenpaar, wobei mindestens eine der Elektroden durch ein Dielektrikum isoliert und wobei durch Anlegen einer Spannung an das Elektrodenpaar durch die Spannungsquelle **2** ein Plasma erzeugt wird. Beispielsweise kann der Plasmagenerator **3** ein Plasma mit einer Intensität erzeugen, welche für eine Desinfektion von Körperbereichen ausgelegt ist und vorzugsweise in Bezug auf Zusammensetzung und/oder Konzentration im gewünschten Bereich liegt (z.B. keine zulässigen Ozonwerte überschreitet).

Werden die Ionen, welche im Plasma erzeugt werden, durch einen Lüfter der Vorrichtung (nicht in Fig. 1 gezeigt) zum lonensensor **4** geführt oder geblasen, werden diese detektiert. Der Ionensensor **4** wird in der Fig. 1 als **SENS** bezeichnet und kann vorzugsweise in Form eines Messfühlers aus einem leitfähigen Material, besonders bevorzugt als Metallstab vorliegen.

Der lonensensor **4** ist mit einer Auswertungseinheit **6** verbunden, welche durch das Symbol **SENS** / **LIMIT** gekennzeichnet ist.

Die Auswertungseinheit **4** erhält ein Messignal des lonensensors **4** über die Detektion der Ionen lonensensor. Sofern ein Plasma mit hinreichende Intensität anhand des Messignals des lonensensors 4 detektiert wird, schaltet die Auswerteeinheit **4** mit einer gewissen Verzögerung den Ausgang am Diagnosestecker ("Plasma-OK") auf 15V und eine LED **15** (z.B. blau) auf der Platine wird eingeschaltet.

Darüber hinaus wird je nach Intensität des Messignal des lonensensors **4,** beispielsweise in Form eines lonenstroms, der Plasmagenerator **3** in seiner Leistung heruntergeregelt.

Der lonensensor **4** kann zu dem Zweck vorzugsweise aus einem Metallstab bestehen, welcher in einer geringen Entfernung zum Plasmagenerator **3** in Strömungsrichtung hinter dieser angebracht ist. Die Ionen, welche an dem Plasmagenerator **3** erzeugt werden, werden durch die Luftströmung zum lonensensor **4,** vorzugsweise in Form eines Metallstabs, transportiert.

Eine beispielhafte Regelung kann wie folgt erfolgen.

Aufgrund der Form der Hochspannungspulse und Erzeugung eines asymmetrischen Wechselfeldes, werden Ionen eines Vorzugzeichens, beispielsweise positive Ionen, vermehrt dem lonensensor **4** in Form eines Metallstabes zugeführt. Somit treffen mehr positive als negative Ionen auf dem lonensensor **4** auf, so dass dieser einen positiven Strom erzeugt (lonenstrom). Dieser Strom kann beispielsweise im Normalfall, d.h. im gewünschten Arbeitsbereich, zwischen 15 nA und 20 nA liegen. Überschreitet das Messignal des lonensensors, hier in Form eines lonenstrom, einen Schwellwert von 15nA, dann wird das Signal "Plasma-OK" gesetzt (blaue LED geht an, Plasma-OK Pin am D/I-Con geht auf 15V).

Übersteigt dieser Strom einen ersten Schwellwert von 15.5nA, wird die Spannungsquelle **2** bzw. der dadurch betriebene Plasmagenerator **3** mit zunehmendem lonenstrom in seiner Frequenz und damit Leistung herunter geregelt. Die Regelung greift mit größerem lonenstrom (d.h. zunehmender Überschreitung) als Messsignal des lonensensors **4** zunehmend stärker ein, so dass die Spannungsquelle **2** bzw. der dadurch betriebene Plasmagenerator **3** ab einem zweiten Schwellwert für die Stromstärke von 22.5 nA auf theoretisch Null herunter geregelt werden würde. Somit ergibt sich ein Gleichgewichtszustand, in welchem der Plasmagenerator derart geregelt wird, dass ein Messsignal des lonensensors **4** in Form eines lonenstrom zuverlässig in einem Arbeitsbereich zwischen 15 nA und 20 nA liegt. Für diesen Arbeitsbereich kann ein Plasma gewährleistet werden, welches beispielweise für die gewünschte Funktion der Vorrichtung zur Desinfektion von Körperbereichen eine hinreichende Intensität aufweist, ohne dass zulässige Grenzwerte für bestimmte Plasmakomponenten, wie beispielsweise Ozon überschritten werden.

Bei abgeschaltetem Lüfter bzw. Gebläse wird eine Asymmetrie, für die auf den Ionensensor 4 auftreffenden Ionen, nicht mehr erreicht. Insbesondere können ohne Lüfter bzw. Gebläse auch langsame Ionen den Ionensensor **4** erreichen und werden nicht weggeblasen. Es erreichen daher genau so viele positive Ionen wie negative Ionen den lonensensor **4** und somit ist der gemessene lonenstrom als Messsignal des lonensensors **4** nahezu Null.

In bevorzugten Ausführungsformen kann der Diagnose- und Interface-Steckverbinder (D/I Con) weiterhin folgende Anschlüsse bzw. Funktionen zur Steuerung der Vorrichtung aufweisen.

Eine Anschluss "24V und PCB-GND" kann für die Versorgungsspannung des Plasmamoduls genutzt werden. Diese Anschlüsse können verwendet werden, um zusätzliche externe Beschaltungen mit Strom zu versorgen. Ebenfalls kann im Falle einer Funktionsüberprüfung (Diagnose) des Moduls die Platine über diese Anschlüsse mit Spannung versorgt werden, die Schaltung also ohne Netzspannung betrieben werden.

Wenn der lonenstrom 15 nA überschreitet, wird ein Anschluss "Plasma OK" auf 15 V gesetzt (mit 10 kOhm Vorwiderstand), sonst 0V. Dieser Anschluss dient vorzugsweise der übergeordneten Elektronik dazu ihr mitzuteilen, dass das Plasma eine hinreichende Intensität hat. Aufgrund der Messsystems und dessen gezwungenermaßen großen Trägheit schaltet dieser Anschluss mit einer gewissen Verzögerung auf 15V (etwa 3 Sekunden nach Einschalten des Plasmas).

Ein Anschluss "PWM-Diag" dient zur Funktionsüberprüfung und Fehlerdiagnose.

Im ungeregelten Betrieb (Eingeschaltetem Plasmagenerator aber abgeschaltetes Gebläse bzw. Lüfter) liefert dieser Anschluss vorzugsweise eine Spannung von 12.7 (+/- 0.9) V. Im normalen Betrieb, also der Plasmagenerator liefert im gewünschten Arbeitsbetrieb einen hinreichenden lonenstrom und der Lüfter bzw. das Gebläse ist eingeschaltet, wird die Frequenz des Plasmagenerators herunter geregelt, womit dessen Spannung absinkt. Im Vergleich zwischen dem Betrieb des Plasmagenerators mit ausgeschaltetem Gebläse zu eingeschaltetem Gebläse sollte die Spannung vorzugweise zwischen 1V und 2V absinken.

**Fig. 2** ist eine Darstellung eines Schaltbildes einer weiteren bevorzugten Ausführungsform der erfindungsgemäßen Vorrichtung **1.**

In der in Fig. 1 gezeigten bevorzugten Ausführungsformen der Vorrichtung ist der lonensensor eine von dem Plasmagenerator separate Messfühler bzw. Metallstab, welcher vorzugsweise in Strömungsrichtung des Luftstromes hinter dem Plasmagenerator **3** angeordnet ist.

In einigen Ausführung kann jedoch auch auf die Bereitstellung eines zum Plasmagenerators **3** separaten Messfühlers bzw. Metallstabs verzichtet werden. Stattdessen kann vorzugsweise eine Gegenelektroden des Plasmagenerators **3** unmittelbar als Messfühler bzw. lonensensor **4** verwendet werden. Zudem diesem Zweck kann es bevorzugt sein die Gegenelektrode vorzugsweise über einen Kondensator an ein Massepotential zu koppeln. Die Fig. 2 illustriert als Schaltbild eine derartige bevorzugte Ausführungsform.

Die Ausgestaltung und Funktion der weiteren Komponenten gelten analog zu obigen Erläuterungen zur Ausführungsform gemäß der Fig. 1.

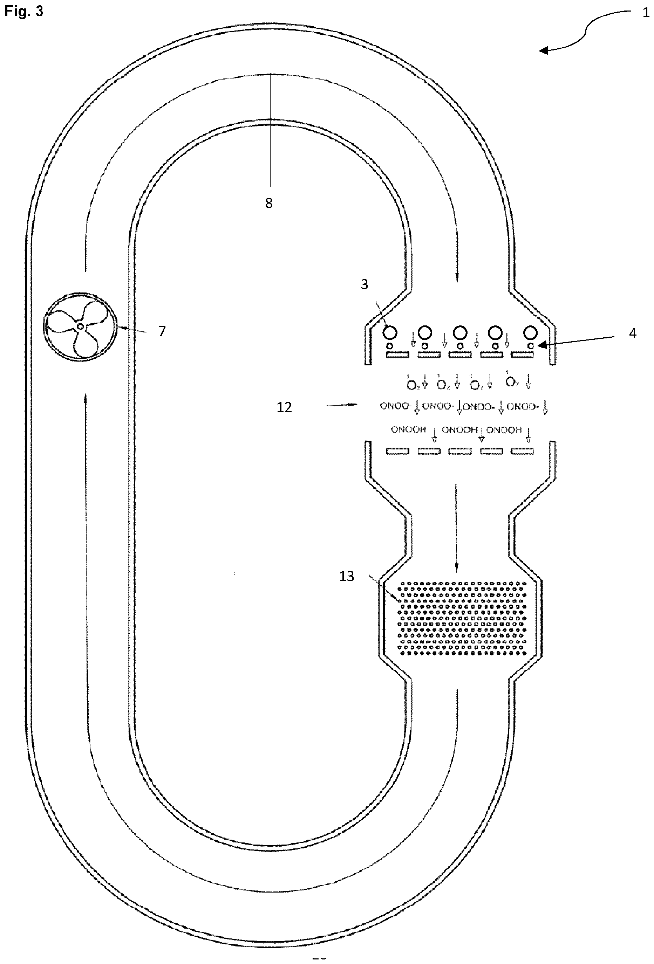

**Fig. 3** ist eine Darstellung einer bevorzugten Vorrichtung **1** zum Desinfizieren und/oder Trocknen von Körperbereichen, bevorzugt Händen.

Die Vorrichtung **1** umfasst einen Lüfter **7,** der Luft aus der Umgebung ansaugt und einen Luftstrom **8** erzeugt. Heizelemente, die nicht abgebildet sind, beheizen die angesaugte Luft. Der Luftstrom **8** läuft über einen Strömungskanal. Des Weiteren wird von der Vorrichtung **1** ein Plasmagenerator **3** umfasst. Der Plasmagenerator **3** befindet sich innerhalb einer Reaktionskammer.

Die Ausgestaltung des Plasmagenerators **3** als Array ist bildhaft präsentiert. Am Plasmagenerator **3** wird eine hohe Wechselspannung angelegt, vorzugsweise in Form von asymmetrischen Hochspannungspulsen durch einen Hochspannungstransformator (nicht gezeigt). Durch das Anlegen der Spannung bildet sich am Plasmagenerator **3** ein sogenanntes kaltes Plasma nach dem Prinzip der dielektrischen Barriereentladung. Dabei können sich reaktive Spezies mit desinfizierender Wirkung, wie reaktive Sauerstoff (ROS)- und Stickstoffspezies (RNS) bilden. Die ROS sind so stabil wie der Singulett-Sauerstoff ¹O₂ und/oder es bilden sich Radikale, die ebenfalls langlebig sind. So entsteht im Plasma vorzugsweise das Superoxid-Radikal O₂·⁻, das mit Stickstoffmonoxid zu reaktiven RNS umfassend Peroxinitriten ONOO- und ONOOH reagiert. Das Singulett-Sauerstoff ¹O₂ und die Peroxinitrite ONOO- und ONOOH sind in der **Fig. 3** ebenfalls abgebildet. Die kleinen Pfeile geben die Richtung dieser reaktiven Spezies an, in die sie transportiert werden. Durch den Luftstrom **8,** der durch den langgezogenen kurvigen Pfeil abgebildet wird, gelangen sie in eine Desinfektionskammer **13.** Innerhalb der Desinfektionskammer **13** können Körperbereiche, insbesondere Hände, zum Trocknen und/oder Desinfizieren eingeführt und gehalten werden.

In der Desinfektionskammer **13** herrscht ein Unterdruck, sodass der Luftstrom weiter zu einem Aktivkohlefilter **9** gelangt. Der Aktivkohlefilter **9** fängt Schadstoffe des Luftstromes ab. Das Druckverhältnis zwischen dem Unterdruck in der Desinfektionskammer **10** und dem Druck in der Umgebung führt dazu, dass keine Schadstoffe in die Umgebung gelangen. Mit der gereinigten Luft beginnt der Prozess von Neuem, sodass sich ein Kreislauf bildet.

Ein lonensensor **4** liegt in Strömungsrichtung des Luftstromes **8** hinter dem Plasmagenerator **3** bzw. der jeweiligen Plasmastäbe angeordnet vor. Der Ionensensor **4** kann vorzugsweise als ein Messfühler aus einem leitfähigen Material, besonders bevorzugt als Metallstab ausgestaltet sein.

In Abhängigkeit der durch das Plasma erzeugten Ionen, welche auf dem lonensensor **4** auftreffen, wird ein Messignal, beispielsweise ein lonenstrom erzeugt. Wie obig erläutert kann mittels eine Auswerteeinheit (nicht gezeigt) die Leistung der Spannungsquelle und mithin des Plasmagenerators **3** reguliert werden, um ein stabiles Plasma mit den gewünschten Eigenschaften zu gewährleisten.

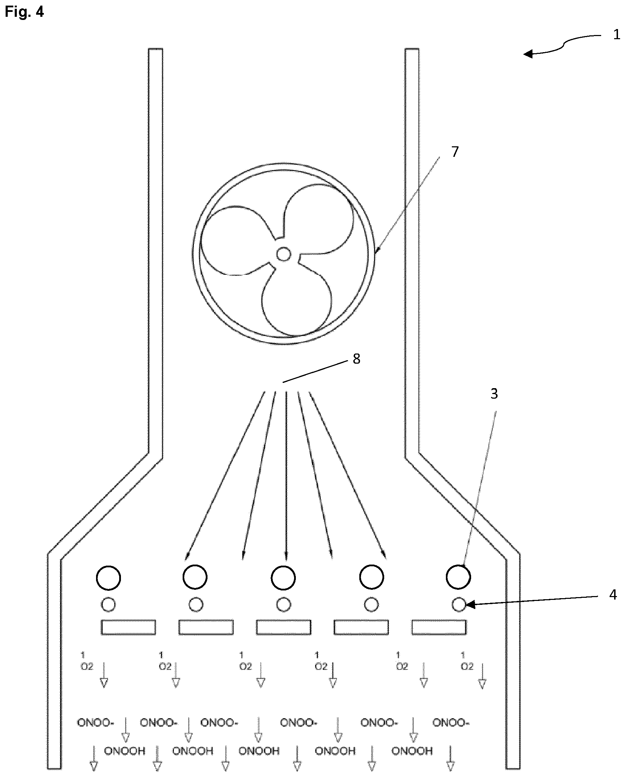

**Fig. 4** ist eine Darstellung einer weiteren bevorzugten Ausführungsform der Vorrichtung **1** zum Desinfizieren und/oder Trocknen von Körperbereichen, bevorzugt Händen. Vorzugsweise ist diese Ausführungsform für Plasmaleistungen ausgelegt, welche die Ozonproduktion reduziert, sodass auf ein Aktivkohlefilter verzichtet werden kann. Anders als in der Ausführungsform in **Fig. 3** wird die Luft durch den Lüfter **7** ausschließlich von der Umgebung angesaugt und nicht in einen Kreislauf überführt, sondern erneut in die Umgebung ausgesetzt. Die angesaugte Luft wird hierbei wieder durch Heizelemente, welche nicht abgebildet sind, beheizt. Über den Strömungskanal gelangt der Luftstrom **8,** der durch die langgezogenen Pfeile visualisiert wird, in Richtung des Plasmagenerators **3.** Das Wirkungsprinzip bleibt zur vorherigen im Hinblick auf **Fig 3** beschriebenen Ausführungsform identisch.

Ein lonensensor **4** liegt auch in dieser Ausführungsform in Strömungsrichtung des Luftstromes **8** hinter dem Plasmagenerator **3** bzw. der jeweiligen Plasmastäbe angeordnet vor, wobei eine Auswerteeinheit (nicht gezeigt) dafür konfiguriert ist, eine Leistung der Spannungsquelle (nicht gezeigt) bzw. Plasmagenerator **3** zu regulieren.

**Fig. 3** zeigt eine bevorzugten Ausführungsform eines Plasmastabes **9,** welcher vom Plasmagenerator **3** umfasst ist. Auf einem dielektrischen Rohr **10** ist ein Draht **12** gewickelt. Innerhalb des dielektrischen Rohres **10** befindet sich ein elektrisch leitender Kern **11.** Der Draht **12** und der Kern **11** bilden ein Elektrodenpaar. Bei Anlegen einer Spannung, vorzugsweise in Form von asymmetrischen Hochspannungspulsen durch einen Hochspannungstransformator (nicht gezeigt) kommt es auf der Außenseite des dielektrischen Rohres **10** nach dem Prinzip der dielektrischen Barriereentladung zur Bildung eines sogenannten kalten Plasma. Dabei bilden sich Ionen und reaktiven Spezies, welche vom Luftstrom **8** weiterbefördert werden.

Wie in **Fig. 3** zu sehen ist, ist der äußere Draht **12** oppelt und gegenläufig entlang der Oberfläche des dielektrischen Rohres**10** gewickelt. Dadurch wird die Induktivität der Wicklung kompensiert. Das Plasma bildet sich dabei nur entlang der dünnen Linie der Drahtwicklung. Der Draht **12** kann mit einem fast beliebigen Wicklungsabstand gewickelt werden, wobei der Abstand zwischen den Windungen bevorzugt bis zu einem 10-fachen Außendurchmesser, bevorzugt zwischen einem 1-fachen und dem 5-fachen, besonders bevorzugt zwischen dem 2-fachen und dem 4-fachen, des dielektrischen Rohres beträgt. Daher lässt sich die Leistung trotz hoher Frequenz und hohem Wechselspannungswert auf ein gewünschtes Maß reduzieren, ohne dabei die Länge des dielektrischen Rohres **10** zu verringern. Die Erzeugung eines Plasmas bzw. reaktiver Spezies kann auf eine größere Strecke, nämlich der Länge des dielektrischen Rohres **10** und auf eine größere Fläche verteilt werden. Damit kann ein großes Strömungsprofil gleichmäßig mit reaktiven Spezies versorgt werden.

Durch das Feedback des lonensensors (nicht gezeigt), welcher mittels einer Auswerteeinheit die Leistung der Spannungsquelle reguliert, werden vorteilhaft auch über eine große Wirkfläche äußerst stabile Bedingungen für die Erzeugung des Plasmas gewährleistet.

### BEZUGSZEICHENLISTE

- 1: Vorrichtung
- 2: Spannungsquelle
- 3: Plasmagenerator
- 4: lonensensor
- 5: Steuereinheit
- 6: Auswerteeinheit
- 7: Lüfter
- 8: Luftstrom
- 9: Plasmastab
- 10: dielektrisches Rohr
- 11: Kern
- 12: gewickelter Draht
- 13: Desinfektionskammer
- 14: Aktivkohlefilter
- 15: Optische Anzeige, vorzugweise LED

## Patentansprüche

1. Vorrichtung (1) zur Erzeugung von Ionen in einem Luftstrom (8) umfassend eine Spannungsquelle (2) und einen Plasmagenerator (3), wobei sich der Plasmagenerator (3) in dem Luftstrom (8) befindet
**dadurch gekennzeichnet, dass** die Vorrichtung (1) einen lonensensor (2) und eine Auswerteeinheit (6) aufweist, wobei die Auswerteeinheit (6) dazu konfiguriert ist in Abhängigkeit eines Messsignals des lonensensors (2) eine Leistung der Spannungsquelle (2) zu regulieren.

2. Vorrichtung (1) gemäß dem vorherigen Anspruch
**dadurch gekennzeichnet, dass** der Plasmagenerator (3) eine Elektrodenpaar umfasst, wobei mindestens eine der Elektroden durch ein Dielektrikum isoliert und wobei durch Anlegen einer Spannung an das Elektrodenpaar durch die Spannungsquelle (2) ein Plasma erzeugt wird.

3. Vorrichtung (1) gemäß einem der vorherigen Ansprüche
**dadurch gekennzeichnet, dass**
die Spannungsquelle (2) einen Transformator, vorzugsweise einen Hochspannungstransformator, umfasst, wobei die Spannungsquelle (2) besonders bevorzugt zur Erzeugung asymmetrischer Hochspannungspulse konfiguriert ist.

4. Vorrichtung (1) gemäß einem der vorherigen Ansprüche
**dadurch gekennzeichnet, dass**
der Plasmagenerator (3) mindestens ein Plasmastab (9) umfasst, welcher ein dielektrisches Rohr (10) mit einem elektrisch leitenden Kern (11) innerhalb des dielektrischen Rohres (10) umfasst, wobei das dielektrische Rohr (10) auf einer Außenseite einen über zu Windungen gewickelten Draht (12) aufweist und wobei der elektrisch leitende Kern (11) mit dem auf der Außenseite gewickelten Draht (12) ein Elektrodenpaar bildet, welches bei Anlegen einer Spannung ein Plasma erzeugt.

5. Vorrichtung (1) gemäß einem der vorherigen Ansprüche
**dadurch gekennzeichnet, dass**
die Vorrichtung (1) einen Lüfter (7) umfasst, welcher zur Erzeugung des Luftstroms (8) konfiguriert ist.

6. Vorrichtung (1) gemäß einem der vorherigen Ansprüche
**dadurch gekennzeichnet, dass**
der lonensensor (4) in Strömungsrichtung des Luftstroms (8) hinter dem Plasmagenerator (3) angeordnet vorliegt.

7. Vorrichtung (1) gemäß einem der vorherigen Ansprüche
**dadurch gekennzeichnet, dass**
der lonensensor (4) einen Messfühler aus einem leitfähigen Material umfasst, wobei der Messfühler vorzugsweise in Form eines Sensorstabes vorliegt und das leitfähige Material vorzugsweise ein Metall ist.

8. Vorrichtung (1) gemäß einem oder mehreren der vorherigen Ansprüche
**dadurch gekennzeichnet, dass**
das Messsignal des lonensensors (4) ein Strom ist.

9. Vorrichtung (1) gemäß einem oder mehreren der vorherigen Ansprüche
**dadurch gekennzeichnet, dass**
die Auswerteeinheit (6) bei Überschreiten eines ersten Schwellwertes des Messignals des lonensensors (4) dazu konfiguriert ist, die Leistung für die Spannungsquelle (2) zu reduzieren, wobei es besonders bevorzugt ist, dass die Leistung für die Spannungsquelle (2) derart in Abhängigkeit von einer Überschreitung des ersten Schwellwertes reduziert wird, dass ab einem zweiten Schwellwert des Messignals des lonensensors (4) die Leistung der Spannungsquelle (2) auf nahezu Null herunter reguliert wird.

10. Vorrichtung (1) gemäß einem oder mehreren der vorherigen Ansprüche
**dadurch gekennzeichnet, dass**
die Auswerteeinheit (6) dafür konfiguriert ist in Abhängigkeit eines Messignals des lonensensors (4) eine Anzeige, vorzugweise eine optische Anzeige, an der Vorrichtung zu aktivieren.
